(19) Europäisches Patentamt
European Patent Office
Office européen des brevets

(11) **EP 4 261 829 A1**

(12) **EUROPEAN PATENT APPLICATION**
published in accordance with Art. 153(4) EPC

(43) Date of publication:
**18.10.2023 Bulletin 2023/42**

(21) Application number: **21903576.3**

(22) Date of filing: **27.08.2021**

(51) International Patent Classification (IPC):
**G16B 40/20** (2019.01)   **G16B 30/10** (2019.01)
**G16B 50/00** (2019.01)

(52) Cooperative Patent Classification (CPC):
**G16B 30/10; G16B 40/20; G16B 50/00**

(86) International application number:
**PCT/KR2021/011553**

(87) International publication number:
**WO 2022/124529 (16.06.2022 Gazette 2022/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.12.2020   KR 20200170138**

(71) Applicant: **Cell & Brain Co., Ltd.
Jeonju-si, Jeollabuk-do 54871 (KR)**

(72) Inventors:
• **SUH, Hae Young**
  **Seongnam-si Gyeonggi-do 13633 (KR)**
• **KIM, Au Jin**
  **Seongnam-si Gyeonggi-do 13633 (KR)**
• **CHANG, Da Young**
  **Yongin-si Gyeonggi-do 16936 (KR)**
• **KIM, Sung Soo**
  **Seoul 05393 (KR)**
• **KIM, Sang Ho**
  **Anyang-si Gyeonggi-do 13979 (KR)**

(74) Representative: **De Clercq & Partners
Edgard Gevaertdreef 10a
9830 Sint-Martens-Latem (BE)**

(54) **GENE INSERTION LOCATION ANALYSIS SYSTEM AND METHOD FOR STEM CELL THERAPEUTIC AGENT HAVING SPECIFIC GENE INSERTED THEREIN**

(57)    A gene insertion location analysis system for a stem cell therapeutic agent having a specific gene inserted therein comprises: a basic information management unit for managing information essential for operating a gene insertion location analysis system for a stem cell therapeutic agent having a specific gene inserted therein; a gene insertion location analysis unit for managing information for the gene insertion location analysis system; and a DB management unit for managing a DB generated or referred by the basic information management unit and gene insertion location analysis unit.

FIG. 1

**Description**

Technical Field

The following description relates to a gene insertion location analysis system and method for a stem cell therapeutic agent having a specific gene inserted therein.

Background Art

Gene therapy, cell therapy, and gene-cell therapy having an inserted gene have been developed as next-generation biopharmaceuticals for the treatment of rare and incurable diseases that cannot be treated with new synthetic drugs.

Gene therapy cannot overcome realistic limitations due to the possibility of causing cancer and technical limitations, and cell therapy uses living cells for treatment. In the early days, the cell therapy was a therapeutic agent for skin regeneration and cartilage defects using skin cells or cartilage cells, but recently, as research on cell therapy (second-generation cell therapy) into which hyperfunctional genes are inserted has been activated rather than simple cell therapy (first-generation cell therapy) targeting tumors and degenerative diseases, the cell therapy is located at the center of development. The cell therapy mainly uses adult stem cells (hematopoietic stem cells or mesenchymal stem cells). The hematopoietic stem cells are cells that constantly produce blood throughout life through proliferation and differentiation, and about 1 million mesenchymal stem cells are present in the bone marrow so as to help the proliferation of hematopoietic stem cells, and known to help the regeneration of various organs out of the bone marrow. Mesenchymal stem cell-based cell therapy is known to be effective in promoting the regeneration of damaged tissue rather than replacing the damaged tissue through differentiation. Since the mesenchymal stem cells alone have proven to be lacking in effectiveness for regenerative therapy, the mesenchymal stem cells are inserted with a hyperfunctional gene and then subcultured to develop cell therapy.

Such an *in vitro* manipulation process (*in vitro* manipulation method - insertion for a hyperfunctional gene, subculture, culture conditions, etc.), the differentiation ability of the cell itself, and the like imply genetic instability. It is discussed that the genetic instability is caused by mechanisms such as mutation, mismatch repair deficiency, and chromosomal instability. The mismatch repair deficiency refers to a genetic hypermutability state of various genes, and is found at high frequency even in microsatellites having short nucleotide sequence repeats even among length mutations. The chromosomal instability affects the number or overall structure of chromosomes, and chromosomal abnormalities may vary for each cell due to differences between cells.

When such a structural defect occurs, the structural defect may be passed on to the next passage cell, and when proliferation is repeated, replication with chromosomal abnormalities may occur. The US FDA determined that it was virtually impossible to confirm all the theoretical risks associated with gene therapy or gene-cell therapy having an inserted gene by considering the durability of cells or genes, off-target effects (problems of editing unwanted genes), etc. with only pre-marketing clinical trials, and thus considered long-term follow-up important under the assumption that post-marketing clinical studies will resolve theoretical risks in the right place at the right time. In the case of a stem cell therapeutic agent (*ex-vivo* therapy) in which a gene is inserted using a chromosomal insertion virus, the risk of genotoxicity has been suggested. For example, there is a case in which leukemia was induced over 6 years after genes such as LMO2, BMI1, CND2, and EVI1 were inserted into 3 to 10 kb of hematopoietic stem cells (HSC). Mesenchymal Stem Cells (MSCs) are stromal cells, and unlike hematopoietic stem cells, the MSCs have been found that there is no risk of genotoxicity because an *in vivo* colonization in which a bacterial species grows more vigorously in the area directly adjacent to the colony of another species *in vivo* and forms a colony does not occur, and there is no persistence in the body. However, in order to verify safety, it is important to analyze a correlation between insertion locations of genes inserted by subculture and chromosome of mesenchymal stem cells infected with the virus into which the gene was inserted. Genetic sequencing is generally performed by collecting mesenchymal stem cells from bone marrow and infecting the cells with viruses (retro, adeno, lenti, etc.), into which the gene is inserted, and then extracting DNA for each subculture, amplifying the extracted DNA by Linear Amplification Mediated-Polymerase Chain Reaction (LAM-PCR), and analyzing the amplified DNA using various Next Generation Sequencing (NGS) platforms, or using the GenomeWalker method. However, existing analysis methods have problems related to sensitivity, reproducibility, accuracy, and presence of harmful genes, and do not actively utilize the latest Information & Communications Technology (ICT) to efficiently analyze a large quantity of sequencing data.

The aforementioned background art is included or obtained by the present inventor in the process of deriving the disclosure of the present application, and cannot necessarily be known art disclosed to the general public prior to the present application.

Disclosure of the Invention

Technical Goals

An aspect of examples is to provide a system capable of preparing various types of analysis reports by first subculturing mesenchymal stem cells collected from the bone marrow for preparing gene and cell therapies and then extracting DNA from a nucleus of the cell after *in vitro* manipulation (infected with a virus having a specific gene inserted therein) and subculturing to secure nucleic sequence analysis data by a Next Generation Sequencing (NGS) technique, and extracting and storing integration sites (start and end locations), quantity, and biotype information of genes for each subculture and each chromosome, and allow organizations (corporations, public institutions, universities, etc.) for developing domestic and foreign gene and cell therapies to operate like each individual system with one system by applying the latest information technology, cloud computing (SaaS: Software as a Service) technique.

Technical goals to be achieved in the examples are not limited to those described above, and other goals not mentioned above are clearly understood by one of ordinary skill in the art from the following description.

Technical Solutions

According to examples, there are disclosed gene insertion location analysis system and analysis method for a stem cell therapeutic agent having a specific gene inserted therein.

The gene insertion location analysis system for the stem cell therapeutic agent having the specific gene inserted therein includes a basic information management unit configured to manage code information, equipment information, operator information, operation information, customer information, cooperator information, parameter information, reference gene information, cancer-causing gene information, and nucleic sequence conversion table information required for the operating of the gene insertion location analysis system for the stem cell therapeutic agent having the specific gene inserted therein, a gene insertion location analysis unit configured to manage order information (including customer orders, operation instructions, and material order information), contract information, project information, gene insertion location analysis information, and project performance result information for the gene insertion location analysis system, and a DB management unit configured to manage a basic information DB (including a company information DB, a user information DB, a reference genome information DB, a COSMIC information DB, an equipment information DB, a manpower information DB, a material information DB, a standard operation information DB, a nucleic sequence conversion table DB, a code information DB, and a parameter information DB), an order information DB, a contract information DB, a project information DB, a gene insertion location information DB, a sequencing information DB, and a project progress information DB generated or referred by the basic information management unit and the gene insertion location analysis unit.

According to an aspect, the basic information management unit may include a basic information management module including a company information management module for managing company information, a user information management module for managing the user information DB, a reference genome information management module for managing the reference genome information DB, a COSMIC information management module for managing the COSMIC information DB, an equipment information management module for managing the equipment information DB, a manpower information management module for managing the manpower information DB, a material information management module for managing the material information DB, a standard operation information management module for managing the standard operation information DB, a nucleic sequence conversion table management module for managing the nucleic sequence conversion table DB, a code information management module for managing the code information DB, and a parameter information management module for managing the parameter information DB.

According to an aspect, the gene insertion location analysis unit includes an order information management module for managing order information, a contract information management module for managing contract information, a project management module for managing project information, a gene insertion location analysis module for managing gene insertion location information, and a project product management module for managing project progress information.

Further, the gene insertion location analysis method for a stem cell therapeutic agent having a specific gene inserted therein includes the steps of a basic information management step of recording basic information required for operating a gene insertion location analysis system for a stem cell therapeutic agent having a specific gene inserted therein in a DB, a project starting step of registering a consultation process with a customer in a project information DB, registering order information in an order information DB, registering contract conditions based on the order information in a contract information DB, registering project information in a project progress DB, and performing a project, a sequencing data processing step of a data pre-processing step for sequencing data generated in the project performing step, a data formalization step, a data annotation registration step, and an analysis data extraction and registration step, a gene insertion location analysis step of a search condition setting step of setting an analysis target, an analysis method selection step, an analysis report confirmation step of confirming the analyzed result, and a step of registering an analysis

EP 4 261 829 A1

result in a DB, and a project termination step of preparing an analysis report to be submitted to the customer, registering a customer feedback, performing total input cost calculation and billing, and terminating the project.

According to an aspect, the project performing step includes a sequencing operation information registration step of registering sequencing operation information in an order information DB and a project progress information DB with respect to the sequencing operation, and in the sequencing operation information registration step, sequencing data is read sequentially from beginning to end, and when the last record is read, the sequencing operation result information is registered in the gene insertion location information DB and the project progress information DB. In addition, in the sequencing operation information registration step, a record where the sequencing data is a multiple of 4 + 1 is read and then redundant data is registered as one unique value, and variable values are separated and registered in a separate record. In addition, in the sequencing operation information registration step, a record where the sequencing data is a multiple of 4 + 2 is read in units of 4 characters and then the corresponding value is converted to binary number in a nucleic sequence conversion table DB. Here, when there is N or U in the values read in units of 4 characters in the sequencing data, or there is no corresponding value in the nucleic sequence conversion table DB, the values are stored in a base information-error table of the gene insertion location information DB. In addition, in the sequencing operation information registration step, a record where the sequencing data is a multiple of 4 + 4 is compressed as a quality score and then stored in a base information-quality score table of the gene insertion location information DB.

According to an aspect, data for analysis of the gene insertion location is generated, analyzed for each subculture, and registered in the gene insertion location information DB, and a correlation between genes by subculture and chromosome is analyzed.

According to an aspect, in the analysis of the gene insertion location, mesenchymal stem cells collected from the bone marrow at the same time and mesenchymal stem cells into which a plurality of genes is inserted may be subcultured under the same conditions, and expression level information may be comparatively analyzed using a t-value technique. Here, the expression level information may comparatively analyze a total expression level by passage, a total number of genes, an expression level by biotype, an expression level by chromosome, the number of genes by chromosome, an expression level by biotype for each chromosome, an expression level by gene, an expression level of the inserted gene, neighboring genes of the inserted gene, and an expression level of the inserted gene.


Effects

According to the examples,
first, it is possible to identify in advance that there is no theoretical risk at the Investigational New Drug (IND) application with respect to a stem cell therapeutic agent having a plurality of genes inserted therein.

Second, it is possible to efficiently compare nucleic sequence data for each subculture of stem cells having a plurality of genes inserted therein with sequencing data of a human body, and to analyze a correlation between genes by subculture and by chromosome.

Third, it is possible to guarantee the reliability by integrally managing overall processes from customer requests for gene insertion location analysis of a stem cell therapeutic agent having a specific gene inserted therein to report preparation for IND application.

Fourth, it is possible to be used by many researchers at home and abroad like an individual system by applying the latest computer technology, Cloud Computing technology and Service as a Software (SaaS) technology.

The effects of the gene insertion location analysis system and analysis method for the stem cell therapeutic agent having the specific gene inserted therein according to the examples are not limited to those mentioned above, and other effects not mentioned will be clearly understood by those skilled in the art from the following description.


Brief Description of Drawings

FIG. 1 is a schematic diagram illustrating a configuration of a gene insertion location analysis system for a stem cell therapeutic agent having a specific gene inserted therein according to an example.
FIG. 2 is a block diagram schematically illustrating an operation flow of submitting a final gene insertion location analysis report from an analysis request of a customer in the gene insertion location analysis system of FIG. 1.
FIG. 3A is a block diagram illustrating a configuration of a gene insertion location analysis system for a stem cell therapeutic agent having a specific gene inserted therein according to an example.
FIG. 3B is a block diagram illustrating a configuration of a basic information management module in FIG. 3A.
FIG. 3C is a block diagram illustrating a configuration of a basic information DB in FIG. 3A.
FIG. 4 is a flowchart schematically illustrating steps of a method of analyzing an insertion location of a gene inserted into a mesenchymal stem cell using a gene insertion location analysis system of a stem cell therapeutic agent having a specific gene inserted therein and submitting a gene insertion location analysis report from a customer request according to an example.

FIG. 5 is a flowchart illustrating a basic information management step in FIG. 4 in more detail.

FIG. 6 is a flowchart illustrating a project starting step in FIG. 4 in more detail.

FIG. 7 is a flowchart illustrating a project performing step in FIG. 6 in more detail.

FIG. 8 is a flowchart illustrating a sequencing operation information registration step in FIG. 7 in more detail.

FIG. 9 is a flowchart illustrating a sequencing data processing step in FIG. 4.

FIG. 10 is a flowchart illustrating a data pre-processing step in FIG. 9 in more detail.

FIG. 11 is a flowchart schematically illustrating a gene insertion location analysis step in FIG. 4.

FIG. 12 is a flowchart schematically illustrating a project termination step in FIG. 4.

FIG. 13A is a diagram illustrating basic information of NGS Data (FastQ) generated in a sequencing operation information registration step in FIG. 7.

FIG. 13B is a block diagram illustrating an entity-relationship diagram (ERD) of a gene insertion location information DB for managing NGS Data (FastQ) of FIG. 13A.

FIG. 14 is a block diagram illustrating an ERD of an entity group of an analysis target table in a gene insertion location information DB generated in an analysis target data registration step in FIG. 10.

FIG. 15A is a nucleic sequence conversion table DB for conversion to binary numbers when there is no blank in nucleic sequence data of FIG. 13B.

FIG. 15B is a nucleic sequence conversion table DB for conversion to binary numbers when there is a blank in nucleic sequence data of FIG. 13B.

Best Mode for Carrying Out the Invention

Hereinafter, examples will be described in detail with reference to the accompanying drawings. However, since various modifications may be made to the examples, the scope of the present disclosure is not limited or restricted by these examples. It should be understood that all modifications, equivalents and substitutes for the examples are included in the scope of the present disclosure.

The terms used in the examples are used for the purpose of description only, and should not be construed to be limited. The singular expression includes the plural expression unless the context clearly dictates otherwise. In the present specification, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which examples pertain. Terms which are defined in a generally used dictionary should be interpreted to have the same meaning as the meaning in the context of the related art, and are not interpreted as ideal or excessively formal meanings unless otherwise defined in the present application.

In addition, in the description with reference to the accompanying drawings, like components designate like reference numerals regardless of reference numerals and a duplicated description thereof will be omitted. In describing the examples, a detailed description of related known technologies will be omitted if it is determined that they unnecessarily make the gist of the examples unclear.

In describing the components of the examples of the present disclosure, terms including first, second, A, B, (a), (b), and the like may be used. These terms are just intended to distinguish the components from other components, and the terms do not limit the nature, sequence, or order of the components. When it is disclosed that any component is "connected", "coupled", or "linked" to other components, it should be understood that the component may be directly connected or linked to other components, but another component may be "connected", "coupled", or "linked" between the respective components.

Components included in any one example and components having a common function will be described using the same names in other examples. Unless otherwise stated, descriptions described in any one example may also be applied to other examples, and detailed descriptions in the overlapping range will be omitted.

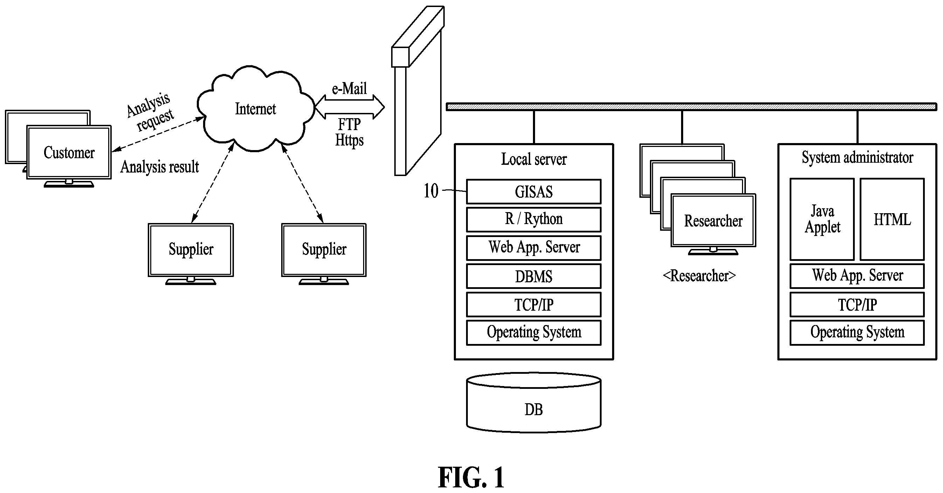

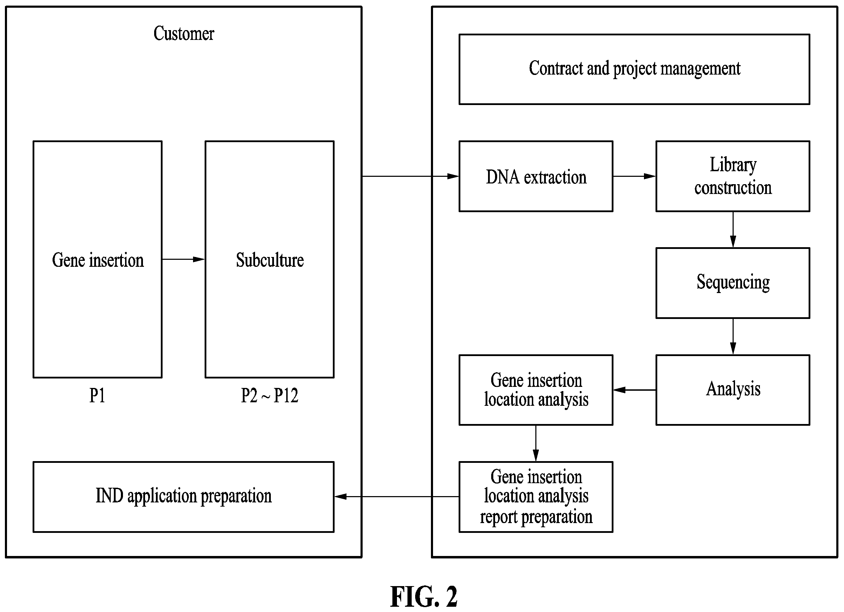

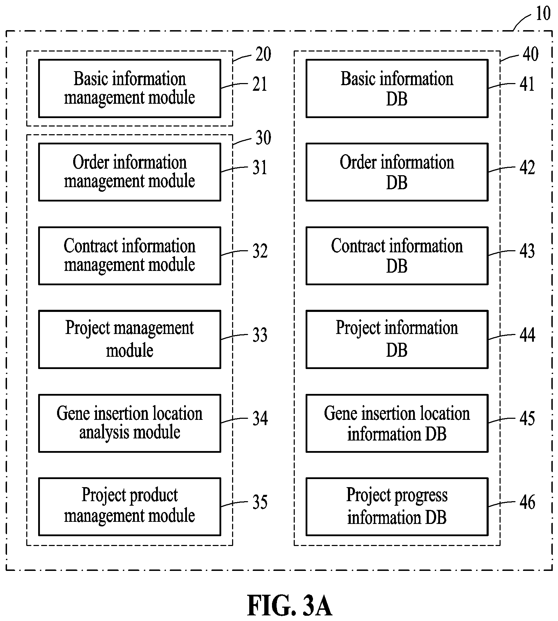

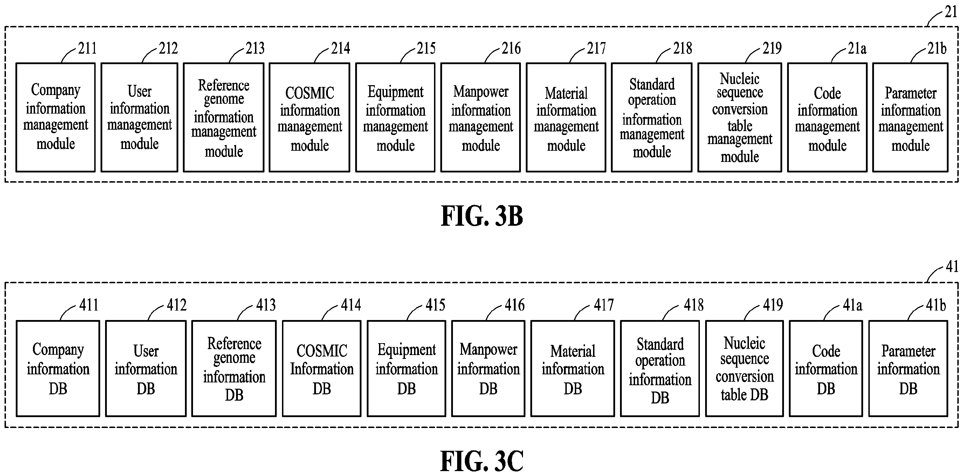

Hereinafter, a gene insertion location analysis system 10 (hereinafter, referred to as the 'gene insertion location analysis system') for a stem cell therapeutic agent having a specific gene inserted therein according to examples and a method thereof will be described with reference to FIGS. 1 to 15B. For reference, FIG. 1 is a schematic diagram for describing an operating concept of a gene insertion location analysis system 10 for a stem cell therapeutic agent having a specific gene inserted therein based on a cloud computing technology according to an example. FIG. 2 is a block diagram schematically illustrating a series of operation flows of making a contract with a customer requesting gene insertion location analysis, taking over stem cells subcultured after gene insertion from a customer, extracting DNA, constructing a library, generating sequencing data, managing and analyzing the quality of sequencing data, analyzing a gene insertion location, preparing a gene insertion location analysis report, and managing a project in the gene insertion location analysis system 10 of FIG. 1. FIG. 3A is a block diagram illustrating a configuration of a gene insertion location analysis system 10 according to an example, FIG. 3B is a block diagram illustrating a configuration of a basic information management module 21 in FIG. 3A, and FIG. 3C is a block diagram illustrating a configuration of a basic information DB 41 in FIG. 3A.

Referring to FIGS. 1 and 2, a gene insertion location analysis system (gene integration site analysis system) 10 may be used like an individual system by many domestic and foreign researchers by applying cloud computing technology and Service as a Software (SaaS) technology. In addition, the gene insertion location analysis system 10 integrally manages overall processes of extracting DNA according to a request of a customer, constructing a library, processing and analyzing sequencing data, performing the analysis of the gene insertion location, and performing an operation of preparing an analysis report to prepare a report for subcultured sequencing investigational new drug (IND) application for stem cells having a specific gene inserted therein requested by a customer asking extract.

FIGS. 3A to 3C, the gene insertion location analysis system 10 is configured by including a basic information management unit 20, a gene insertion location analysis unit 30, and a DB management unit 40.

The basic information management unit 20 is configured by including a basic information management module 21 that manages basic information for the operating of the gene insertion location analysis system 10. The basic information management module 21 includes a company information management module 211 for managing a company information DB 411, a user information management module 212 for managing a user information DB 212, a reference genome information management module 213 for managing a reference genome (human genome (GRCh/hg38)) information DB 413, a COSMIC information management module 214 for managing a catalog of somatic mutations in cancer (COSMIC) information DB 414 containing information on somatic mutations related to cancer, and an equipment information management module 215 for managing an equipment information DB 415 performing an operation. In addition, the basic information management module 21 includes a manpower information management module 216 for managing a manpower information DB 416, a material information management module 217 for managing a material information DB 417, a standard operation information management module 218 that manages a standard operation information DB 418 necessary for performing various operations (DNA extraction, library construction, LAM-PCR amplification, NGS sequencing, etc.) to acquire nucleic sequence data of stem cells having a specific gene inserted therein, a nucleic sequence conversion table management module 219 for managing a nucleic sequence conversion table DB 419 for converting nucleic sequence data into binary numbers, a code information management module 21a for managing a code information DB 41a, and a parameter information management module 21b for managing a parameter information DB 41b.

The gene insertion location analysis unit 30 generates orders based on a customer request, registers contract information after signing a contract and starts project management, performs gene insertion location analysis through DNA extraction, library construction, LAM-PCR amplification, and NGS analysis by taking over cells having a gene inserted therein from the customer, prepares a final report to submit the final report to the customer, and performs the termination of the project. The gene insertion location analysis unit 30 is configured by including an order information management module 31 that manages various kinds of order information (customer order, DNA extraction operation order, library construction order, and sequencing order), a contract information management module 32 for managing contract information with the customer, a project management module 33 for managing project information to fulfill the contract of the customer, an analysis module 34 that analyzes the gene insertion location using sequencing data, as a sequencing order result, and a project product management module 35 for submitting the analysis results to the customer and terminating the project.

The order information management module 31 registers order information according to contract conditions confirmed through consultation with the customer, operation order information such as self-production or outsourcing production (DNA extraction operation, library construction, LAM-PCR amplification, NGS sequencing), and purchase order information of required materials in an order information DB 42. The contract information management module 32 registers contract information confirmed by consultation with the customer in a contract information DB 43. The project management module 33 registers project information linked to order information and contract information of the customer in a project information DB 44. The gene insertion analysis module 34 extracts NGS sequencing data and data for gene insertion analysis and registers the extracted data in a gene insertion location information DB 45. The project product management module 35 registers project progress information and gene insertion location analysis results in a project progress information DB 46.

The DB management unit 40 is configured by including the DB management unit 40 including the basic information DB 41, the order information DB 42, the contract information DB 43, the project information DB 44, the gene insertion location information DB 45, and the project progress information DB 46.

The basic information DB 41 is configured by including the company information DB 411, the user information DB 412, the reference genome information DB 413, the COSMIC information DB 414, the equipment information DB 415, the manpower information DB 416, the material information DB 417, the standard operation information DB 418, the code information DB 41a, and the parameter information DB 41b, in which data generated or referred by each module of the basic information management unit 20 and the gene insertion location analysis unit 30 is managed by the database management system (DBMS).

Here, the company information DB 411 includes a business registration number or unique number, a serial number, an organization name, a representative phone number, a fax number, an address, a representative name, etc. of an operating company and a customer (company, public institution, university, etc.). The user information DB 412 includes names, passwords, contact information (cell phone, office, fax, etc.), e-mail addresses, position codes, authority codes, e-mail reception, SMS text reception, and the like. The reference genome information DB 413 includes information on types and functions of human genes identified through the human genome project (HGP) and may refer to a DB managed by National Center for Biotechnology Information (NCBI) in the US or copy the DB, store the copied DB in a local computer, and refer to the stored DB. The COSMIC information DB 414 includes information such as gene name, ID number of the Entrez DB, gene location (start and end locations of chromosomal integration site), and roles in cancer. The equipment information DB 415 includes information such as an equipment number, an equipment name, an equipment specification, an equipment maker, a purchase price, a unit price per hour, and the like. The manpower information DB 416 includes required capability information of an operator performing an operation, unit price information per hour, and the like. The material information DB 417 includes a material number, a material name, an input unit, a unit price, supplier code information, etc. of the material corresponding to the required material information of the standard operation information DB 418. The standard operation information DB 418 includes an operation number, an operation name, and an operation time that manage various operation information for analysis of gene insertion location information, equipment information to perform the operation, required material information, and required capacity information of the operator performing the operation, before and after operation information, etc. The nucleic sequence conversion table DB 419 may convert 4-byte (32-bit) characters into variable bit (1 to 8-bit) numbers as a conversion table for efficiently storing data of nucleic sequences (sequentially listing nucleobases (A (adenine), T (thymine), G (guanine), C (cytosine)), which are one of components of a basic unit nucleotide of DNA) of a genome. The code information DB 41a includes a standard operation information number, a standard equipment usage time, a standard material code, a standard material requirement, a standard labor cost code, a standard operation time, standard operation unit price information, and the like. The code information DB includes various code information. The parameter information DB 41b includes information about a Noise reference value, a summation interval of the same gene, a sequencing analysis volume, a cell type, an infection virus type, an inserted gene type, a range of inaccuracy (Noise Data) of mapping the front and back parts of gene insertion location (Integration Site), a predetermined range value that is considered the same site when a transcription site of the same gene is within a predetermined range, sequencing assay values (1 G, 3 G, 10 G, etc.) when targeting a Pooled Sample, and the like.

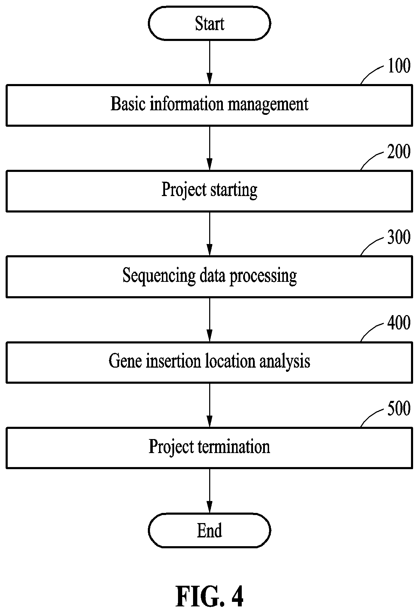

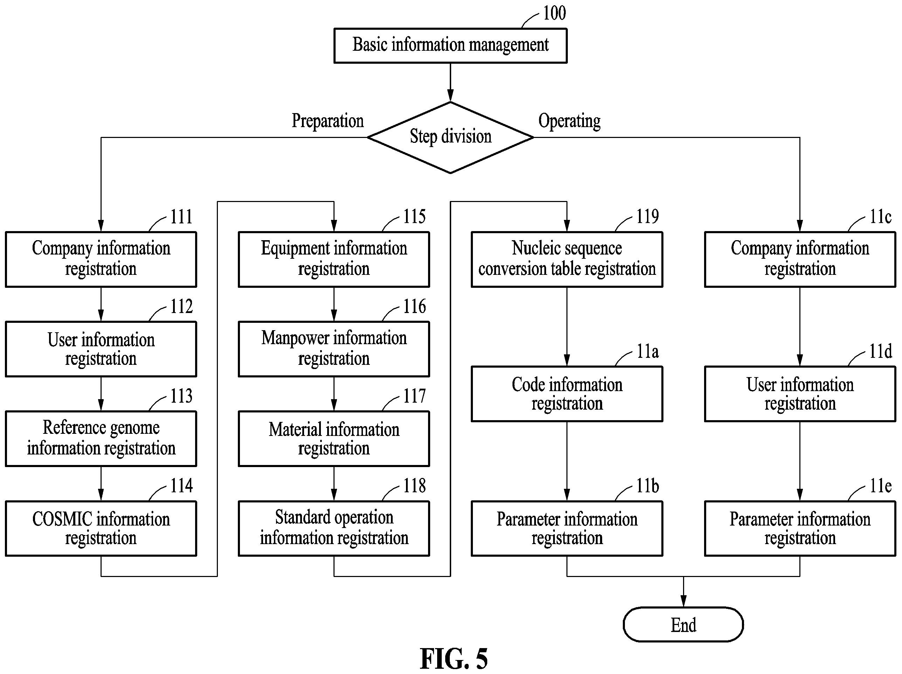

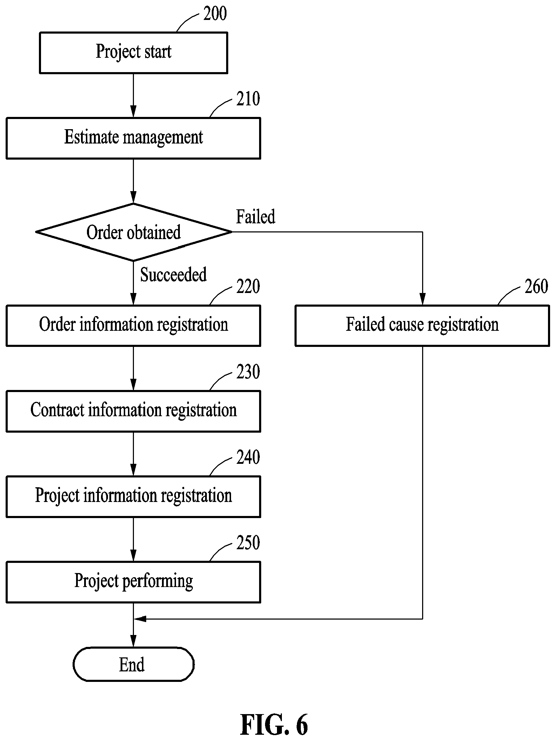

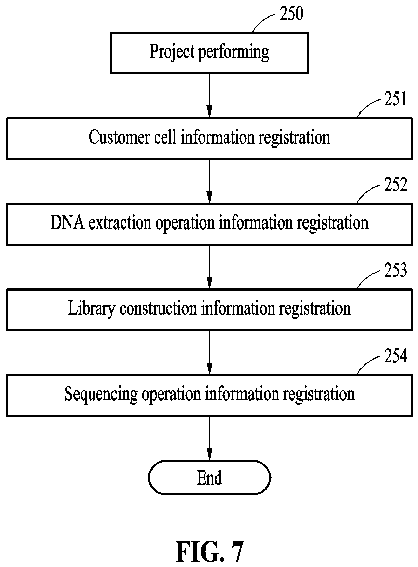

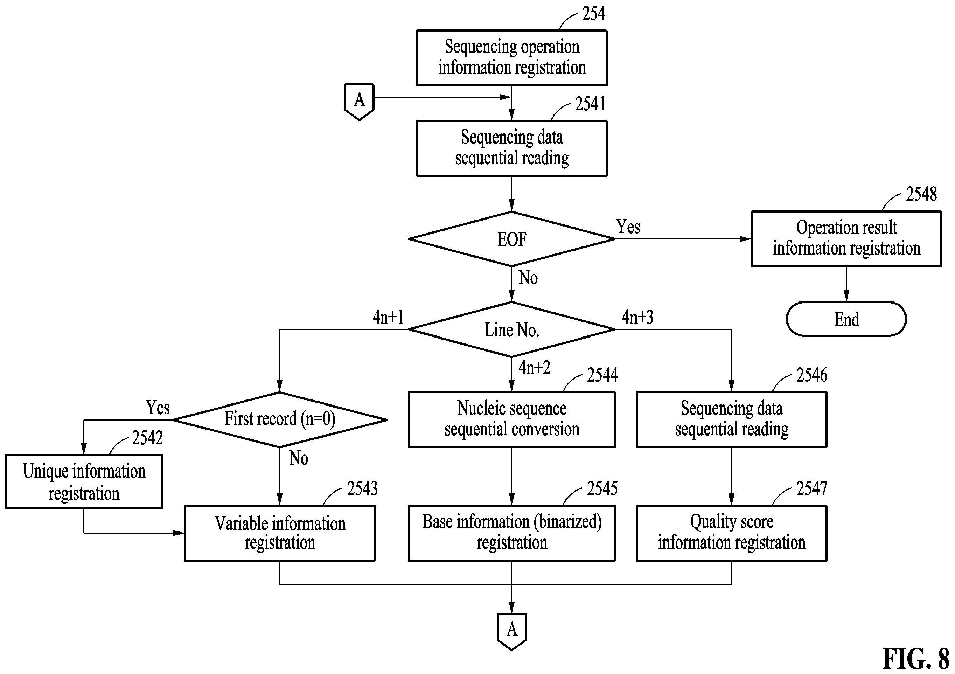

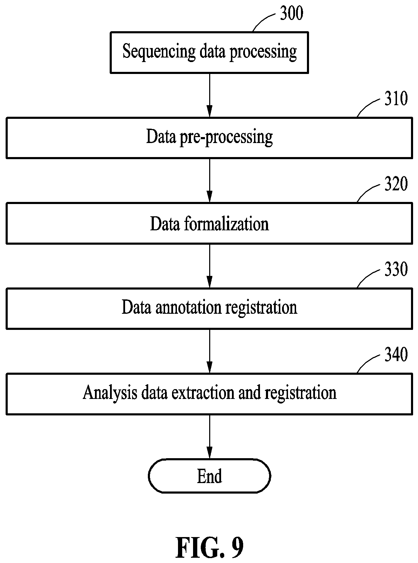

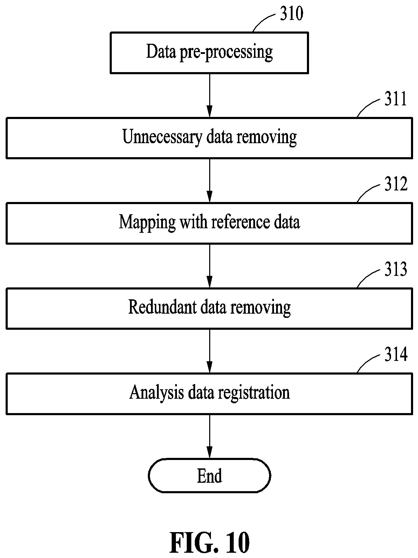

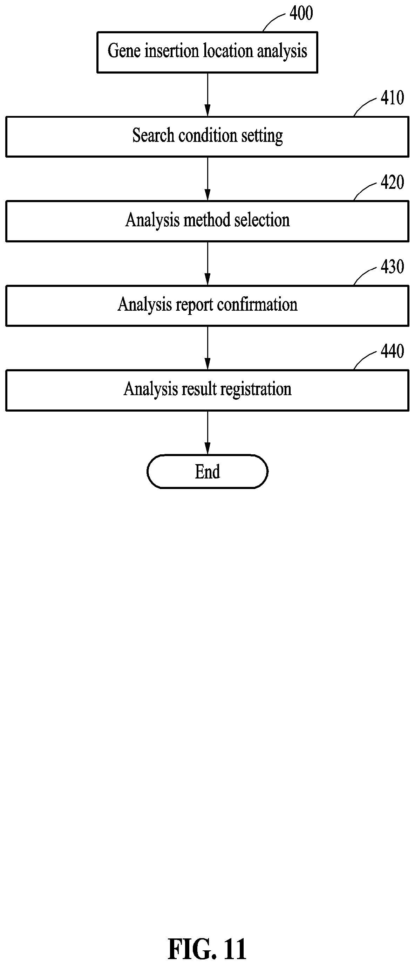

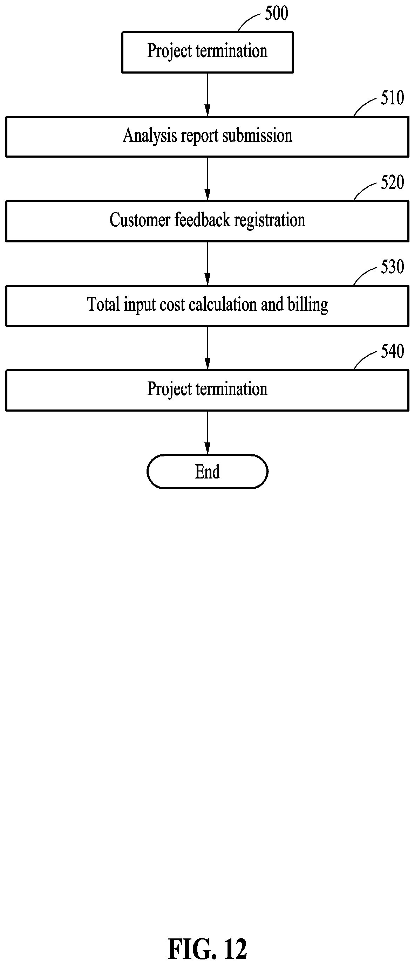

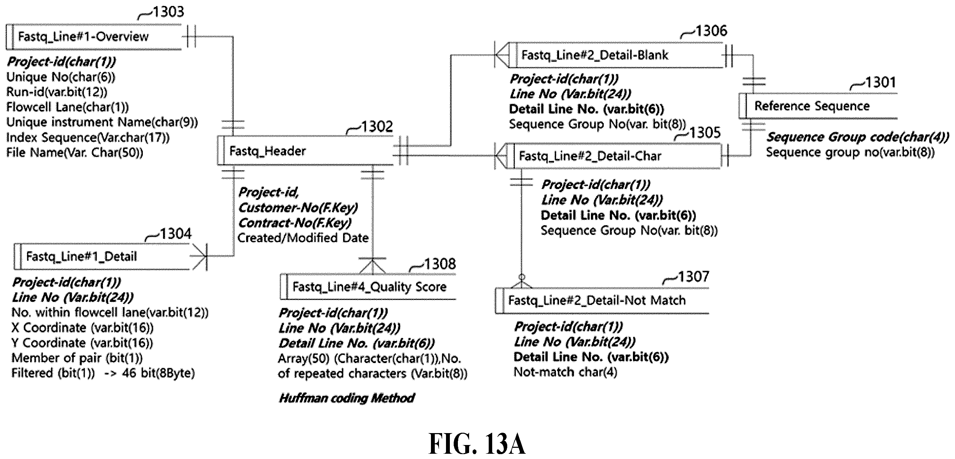

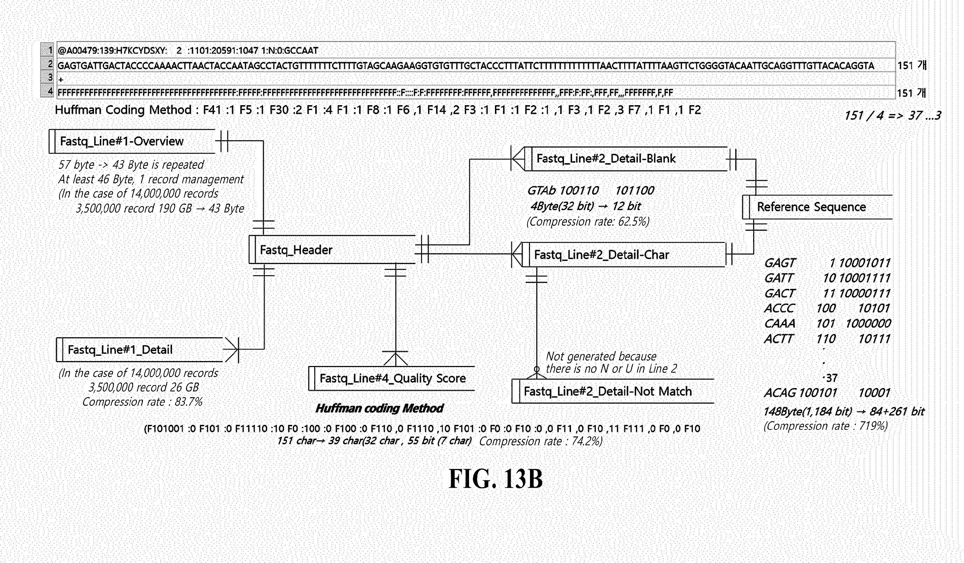

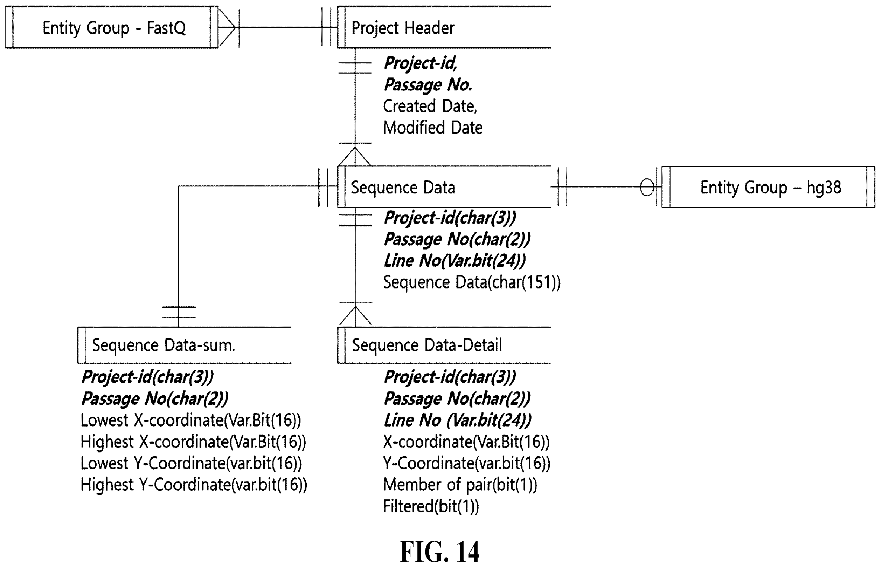

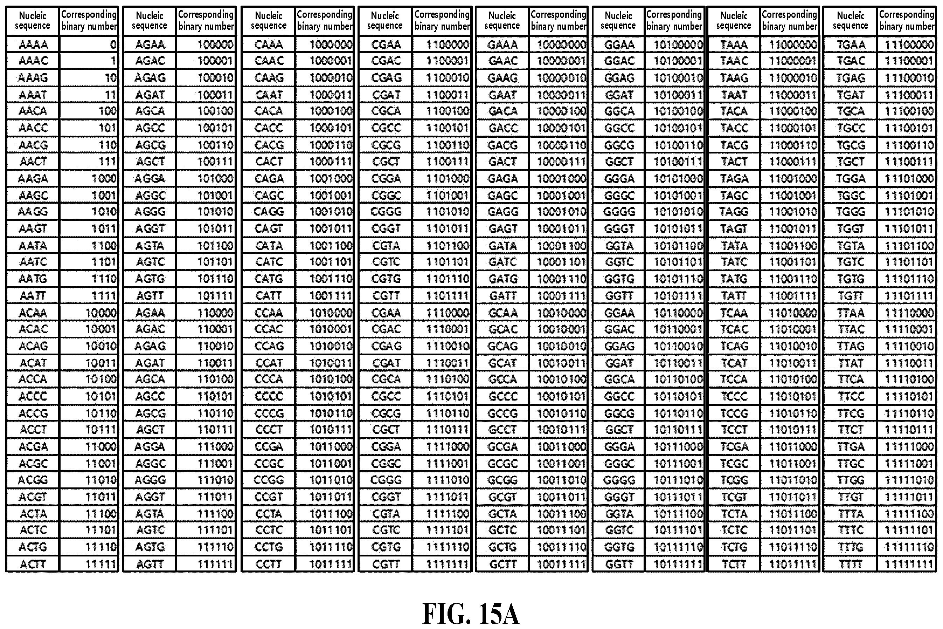

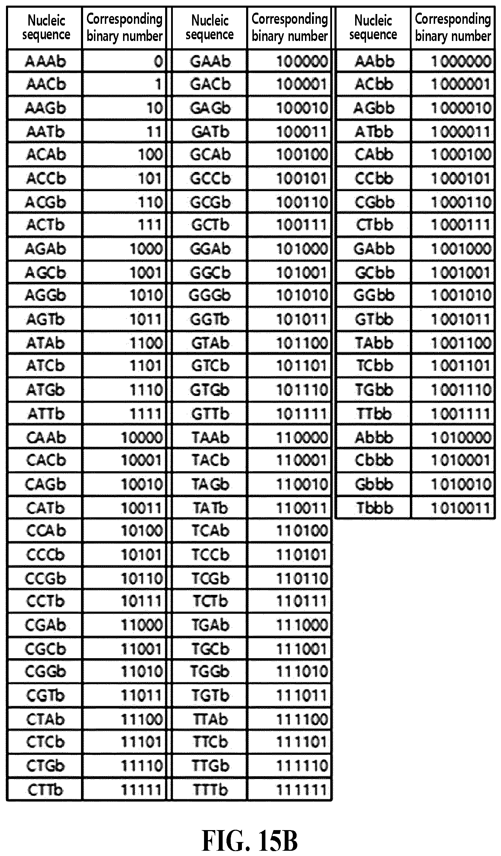

Hereinafter, a method for analyzing insertion locations of genes by subculture and by chromosome of a stem cell therapeutic agent infected with a virus having a specific gene inserted therein using the gene insertion location analysis system 10 according to examples will be described with reference to FIGS. 4 to 15B. For reference, FIG. 4 is a flowchart schematically illustrating a method for analyzing a gene insertion location of stem cells having a specific gene inserted therein by a request of the customer and submitting the results to the customer using the gene insertion location analysis system 10 in FIG. 3A. FIG. 5 is a flowchart illustrating a basic information management step 100 in FIG. 4. FIG. 6 is a flowchart illustrating a project starting step 200 in FIG. 4, FIG. 7 is a flowchart illustrating a project performing step 250 in FIG. 6, and FIG. 8 is a flowchart illustrating a sequencing operation information registration step 254 in FIG. 7. FIG. 9 is a flowchart illustrating a sequencing data processing step 300 in FIG. 4 and FIG. 10 is a flowchart illustrating a data pre-processing step 310 in FIG. 9. FIG. 11 is a flowchart illustrating a gene insertion location analysis step 400 in FIG. 4. FIG. 12 is a flowchart illustrating a project termination step 500 in FIG. 4. FIG. 13A is a diagram illustrating basic information of NGS Data (FastQ) generated in a sequencing operation information registration step 254 in FIG. 7 and FIG. 13B is a block diagram illustrating an entity-relationship diagram (ERD) of a gene insertion location information DB for managing NGS Data (FastQ) of FIG. 13A. FIG. 14 is a block diagram illustrating an ERD of an entity group of an analysis target table in a gene insertion location information DB generated in an analysis target data registration step 314 in FIG. 10. FIGS. 15A and 15B are nucleic sequence conversion table DBs for converting nucleic sequence data of FIG. 13B to binary numbers, in which FIG. 15A is a DB when there is no blank and FIG. 15B is a DB when there is blank.

First, in the basic information management step 100, basic information is recorded in each DB.

Specifically, referring to FIG. 5, when a step division is a preparation step, the basic information management step 100 includes steps of registering company information operating the gene insertion location analysis system 10 in the company information DB 411 by the company information management module 211 in step 111, registering user information belonging to the operating company in the user information DB 412 by the user information management module 212 in step 112, registering reference genome information in the reference genome information DB 413 by the reference genome information management module 213 in step 113, registering list COSMIC information of genes containing information on somatic mutations related to cancer in the COSMIC information DB 414 by the COSMIC information management module 214 in step 114, and registering information on equipment that performs various operations for acquiring nucleic sequence data of stem cells having a specific gene inserted therein in the equipment information DB 415 by the equipment information management module 215 in step 115. In addition, the basic information management step 100 includes steps of registering manpower information (required capacity, unit price per hour, etc.) to perform the corresponding operation in the manpower information DB 416 by the manpower information management module 216 in step 116, registering material information required for the corresponding operation in the material information DB 417 by the material information management module 217 in step 117, registering standard operation information of the corresponding operation in the standard operation information DB 418 by the standard operation information management module 218 in step 118, registering a nucleic sequence conversion table in the nucleic sequence conversion table DB 419 by the nucleic sequence conversion table management module 219 to compress sequence information in step 119, registering code information necessary for system operating in the code information DB 41a by the code information management module 21a in step 11a, and registering various parameters required for system operating in the parameter information DB 41b by the parameter information management module 21b in step 11b.

In addition, when the step division is an operating step, the basic information management step 100 includes steps of registering a new customer to be found in the company information DB 411 by the company information management module 211 in step 11c, registering user information belonging to the customer company in the user information DB 412 by the user information management module 212 in step 11d, and registering parameter information that meets the customer's request in the parameter information DB 41b by the parameter information management module 21b in step 11e.

Referring back to FIG. 4, after the basic information management step 100, the project starting step 200 is performed.

Referring to FIG. 6, the project starting step 200 includes registering a consultation process with the customer in the project information DB 44 using estimate management in step 210. When an order is concluded and obtained as the consultation result, the project starting step 200 includes steps of registering order information in the order information DB 42 in step 220, registering contract conditions based on the order information in the contract information DB 43 in step 230, registering the project information in the project progress DB 44 for management by subculture of the customer in step 240, and performing a project in step 250.

In addition, referring to FIG. 7, the project performing step 250 includes steps of taking over and registering subcultured cells and related information of the customer in the project progress information DB 46 in step 251, registering DNA extraction operation order information in the order information DB 42 and the project progress information DB 46 with respect to the DNA extraction operation in step 252, registering library construction operation order information in the order information DB 42 and the project progress information DB 46 with respect to the library construction operation in step 253, and registering sequencing operation information in the order information DB 42 and the project progress information DB 46 with respect to the sequencing operation in step 254.

In addition, referring to FIG. 8, the sequencing operation information registration step 254 includes steps of sequentially reading the sequencing data (FastQ) submitted by the NGS sequencing operator along with the operation results from beginning to end in step 2541, and registering the sequencing operation result information in the gene insertion location information DB 45 and the project progress information DB 46 when the last record is read in step 2548.

If the record of the sequencing data read in the sequencing data sequential reading in step 2541) is a multiple of 4 + 1 and is a first record (n = 0), since the record is registered in a header table (FastQHeader) 1302 and corresponds to a lead (first line of FIG. 13B) of the sequencing data, unique values (unique information) of the header information are extracted and registered in a unique information table (FastQ_Line#1-Overview) 1303 of the gene insertion location information DB 45 in step 2542, and variable values (variable information) of the header information are registered in a variable information table (FastQ_Line#1-Detail) 1304 of the gene insertion location information DB 45 in step 2543, and the next record is read in step 2541.

In addition, since when the record of the sequencing data read in the sequencing data sequential reading in step 2541 is a multiple of 4 + 2, the record is nucleic sequence data, 4 characters (4 bytes) are read, and the corresponding value (variable binary number, 1 to 8 bits) is confirmed in a Reference Sequence table 1301 of the nucleic sequence conversion table DB 419. For example, referring to FIG. 15A, the binary number is 0 in the case of a nucleic sequence of AAAA, the binary number is 1 in the case of AAAC, the binary number is 1111110 in the case of the nucleic sequence of TTTG, and the binary number is 11111111 in the case of the nucleic sequence of TTTT. The confirmed value is registered in a base information table (FastQ_Line#2_Detail-Char) 1305 of the gene insertion location information DB 45, and the next 4 bytes are processed. When processing in the same manner, as illustrated in FIG. 13B, 151 characters are read and 148 characters (1,184 bits) are converted into location information (84 bits) and conversion information (261 bits), resulting in a compression rate of 71.9%. Also, when the data read by 4 bytes has 1 to 3 blanks, the corresponding value (variable binary number, 1 to 8 bits) is confirmed in the reference sequence table 1301 of the nucleic sequence conversion table DB 419, and the corresponding binary number is assigned with reference to the nucleic sequence conversion table of FIG. 15B. For example, AAAb corresponds to binary 0, AACb corresponds to binary 1, TTTb corresponds to binary 111111, AAbb corresponds to binary 1000000, and Tbbb corresponds to 1010011. As illustrated in FIG. 13B, the compression rate is 62.5% because the remaining 3 characters (1 blank character, 4 bytes, 32 bits) are managed as location information (6 bits) and conversion information (6 bits) of 12 bits. The values confirmed above are registered in the base information table (FastQ_Line#2_Detail-Blank) 1306 of the gene insertion location information DB 45, and the next 4 bytes are processed. In addition, if the read data includes N or U, it is error information, and thus, the error information is registered in a base information-error table (FastQ_Line#2_Detail-Not Match) 1307 of the gene insertion location information DB (45) in step 2545 and the next record is read in step 2541. Here, in the case of FIG. 13B, there is no error data and thus, it is not registered.

In addition, if the record of the sequencing data read in the sequencing data sequential reading in step 2541 is a multiple of 4 + 3, it indicates a simple connection (a value is "+"), and thus, the next record is read in step 2546.

In addition, when the record of the sequencing data read in the sequencing data sequential reading in step 2541 is a multiple of 4 + 4, the record means a value of Quality Score, and thus, the record is compressed by applying a data compression method (Huffman Coding Method) and registered in a base information-quality score table (FastQ_Line#4_Quality Score) 1308 of the gene insertion location information DB 45 in step 2547, and the next record is read in step 2541. For example, when converted by the Huffman Coding Method as shown in FIG. 13B, 151 characters (1208 bits) are compressed into 39 characters and 55 bits (7 characters), and thus, the compression rate is 74.2%.

Referring back to FIG. 4, after the project starting step 200, a sequencing data processing step is performed 300.

Referring to FIG. 9, the sequencing data processing step 300 includes a data pre-processing step 310, a data formalization step 320, a data annotation registration step 330, and an analysis data extraction and registration step 340.

Referring to FIG. 10, the data pre-processing step 310 includes an unnecessary data removing step 311, a step of mapping with reference data 312, a redundant data removing step 313, and an analysis target data registration step 314.

Here, the sequencing data processing step 300 is a general genome sequencing pipeline, which is used for analysis of the extent to which genome expression is affected by a specific environment, analysis of genomes causing diseases, and the like. However, since the example analyzes stem cells having a specific hyperfunctional gene inserted therein, the insertion location correlation analysis of genes for each subculture and each chromosome is necessarily performed, and thus, the unnecessary data removing step 311 and the redundant data removing step 313 of the data pre-processing step 310 may be omitted.

In addition, in the reference data mapping step 312, while the sequencing operation information registration step 254 is performed in reverse order, a data binarized part (4n + 2 Line) is converted into original data, and genetic information mapped with the reference genome information DB 413 is identified using the converted original data in step 312). Then, redundant data among the identified genetic information is removed in step 313 or the analysis target data is registered in the analysis target data table of the gene insertion location information DB 45 without removing the redundant data in step 314.

In the data formalization step 320, a merging step is performed when the data are distributed in the front and back parts of a specific gene insertion location (Integration Site) of a specific gene in step 320.

In the data annotation registration step 330, operations such as associating with neighboring genes, performing gene ontology analysis and genomic feature association analysis, and associating peak values with gene expression data are performed.

In the analysis data extraction and registration step 340, data for gene insertion location analysis is generated and registered in the analysis target data table of the gene insertion location information DB 45.

Referring back to FIG. 4, after the sequencing data processing step 300, a gene insertion location analysis step is performed in step 400.

Referring to FIG. 11, the gene insertion location analysis step 400 includes a search condition setting step 410, an analysis method selection step 420, an analysis report confirmation step 430, and an analysis result registration step 440.

The search condition setting step 410 is a step of determining condition values for searching for the gene insertion location information DB 45 generated in the sequencing data processing step 300. That is, search conditions are set to be customer information, project information, and subculture information for searching all passages or a specific passage.

Next, an analysis method is determined in step 420 after searching the data using the set search conditions, a searched report confirming step 430 is performed by executing the determined analysis method, and the analysis result is registered in the project progress information DB 46 in step 440.

Here, in the analysis of the gene insertion location by subculture, the mesenchymal stem cells collected from the bone marrow at the same time and the mesenchymal stem cells into which a plurality of genes were inserted were subcultured under the same conditions, and expression level information was comparatively analyzed using a t-value technique. Here, the expression level information includes a total expression level by passage, a total number of genes, an expression level by biotype, an expression level by chromosome, the number of genes by chromosome, an expression level by biotype for each chromosome, an expression level by gene, an expression level of the inserted gene, neighboring genes of the inserted gene, an expression level of the inserted gene, and the like. The t-value technique compares average values between the two sample groups as shown in Equation (1) below.

$$t = \frac{\overline{X_1} - \overline{X_2}}{s_{\overline{X_1} - \overline{X_2}}} \quad \cdots\cdots\cdots\cdots\cdots\cdots\cdots\cdots\cdots\cdots\cdots (1)$$

Here, t is a statistical indicator of a difference in sample means, $\overline{X_1} - \overline{X_2}$ is a difference in means between two sample groups, and $s_{\overline{X_1} - \overline{X_2}}$ is the uncertainty about the difference in means between the two sample groups.

The uncertainty may be represented by Equation (2) below.

$$s_{\overline{X_1} - \overline{X_2}} = \sqrt{Var[\overline{X_1} - \overline{X_2}]} = \frac{s_1^2}{n_1} + \frac{s_2^2}{n_2} \quad \cdots\cdots (2)$$

Here, s1 and s2 are standard deviations of each sample, and n1 and n2 are the number of each sample.

Therefore, from Equations (1) and (2), Equation (1) may be represented by the following Equation (3).

$$t = \frac{\overline{X_1} - \overline{X_2}}{\sqrt{\frac{s_1^2}{n_1} + \frac{s_2^2}{n_2}}} \quad \cdots\cdots\cdots\cdots\cdots\cdots\cdots\cdots\cdots\cdots\cdots (3)$$

Meanwhile, assuming that n1 and n2 of the two sample groups are the same and the variances are the same, Equation (3) may be represented by the following Equation (4).

$$t = \frac{\overline{X_1} - \overline{X_2}}{s_p \sqrt{\frac{2}{n_2}}} \quad \cdots\cdots\cdots\cdots\cdots\cdots\cdots\cdots\cdots\cdots\cdots (4)$$

$$s_p = \sqrt{\frac{s_1^2 + s_2^2}{2}}$$

Here, $S_p$ is a pooled standard deviation,

Meanwhile, assuming that n1 and n2 of the two sample groups are different, but the variances are the same, Equation (3) may be represented by the following Equation (5).

$$t = \frac{\overline{X_1} - \overline{X_2}}{sp\sqrt{\dfrac{1}{n_1} + \dfrac{2}{n_2}}} \dots\dots\dots\dots\dots\dots\dots\dots\dots\dots\dots\dots (5)$$

Here,

$$s_P = \sqrt{\frac{(n_1 - 1)s_1^2 + (n_2 - 1)s_2^2}{n_1 + n_2 - 2}}$$

Referring back to FIG. 4, after the gene insertion location analysis step 400, the project is terminated in step 500.

Referring to FIG. 12, the project termination step 500 includes an analysis report submission step 510, a customer feedback registration step 520, a total input cost calculation and billing step 430, and a project termination step 540.

In the analysis report submission step 510, referring to the project progress information DB 46 registered in the gene insertion location analysis step 400, a gene insertion location analysis report of the stem cell therapeutic agent having a specific gene inserted therein is prepared as a PDF file and delivered to the customer.

Next, the feedback of the customer on the analysis report is received and registered in the project progress information DB 46 in step 520. In addition, when the feedback of the customer is received, referring to the operation order DB 42 of the order information 42, a total input cost is calculated by confirming operation time, required materials, input manpower, time information, and the like, registered in the project progress information DB 46, and billed to the customer in step 430. In addition, standard cost information is compared with the total input cost calculated in the total input cost calculation and billing step to calculate the profit and loss, and registered in the project progress information DB 46, and the project is terminated in step 540.

As described above, although the examples have been described by the restricted drawings, various technical modifications and variations can be applied on the basis of the examples by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result can be achieved.

Therefore, other implementations, other examples, and equivalents to the appended claims fall within the scope of the claims to be described below.

## Claims

1. A gene insertion location analysis system for a stem cell therapeutic agent having a specific gene inserted therein, the gene insertion location analysis system comprising:

    a basic information management unit configured to manage code information, equipment information, operator information, operation information, customer information, cooperator information, parameter information, reference gene information, cancer-causing gene information, and nucleic sequence conversion table information required for operating of the gene insertion location analysis system for the stem cell therapeutic agent having the specific gene inserted therein;

    a gene insertion location analysis unit configured to manage order information (comprising customer orders, operation instructions, and material order information), contract information, project information, gene insertion location analysis information, and project performance result information for the gene insertion location analysis system; and

    a DB management unit configured to manage a basic information DB (comprising a company information DB, a user information DB, a reference genome information DB, a COSMIC information DB, an equipment information DB, a manpower information DB, a material information DB, a standard operation information DB, a nucleic sequence conversion table DB, a code information DB, and a parameter information DB), an order information DB, a contract information DB, a project information DB, a gene insertion location information DB, a sequencing information DB, and a project progress information DB generated or referred by the basic information management unit and the gene insertion location analysis unit.

2. The gene insertion location analysis system of claim 1, wherein the basic information management unit comprises a basic information management module comprising a company information management module for managing company information, a user information management module for managing the user information DB, a reference genome information management module for managing the reference genome information DB, a COSMIC information management module for managing the COSMIC information DB, an equipment information management module for managing the equipment information DB, a manpower information management module for managing the manpower information DB, a material information management module for managing the material information DB, a standard operation information management module for managing the standard operation information DB, a nucleic sequence conversion table management module for managing the nucleic sequence conversion table DB, a code information management module for managing the code information DB, and a parameter information management module for managing the parameter information DB.

3. The gene insertion location analysis system of claim 1, wherein the gene insertion location analysis unit comprises an order information management module for managing order information, a contract information management module for managing contract information, a project management module for managing project information, a gene insertion location analysis module for managing gene insertion location information, and a project product management module for managing project progress information.

4. A gene insertion location analysis method for a stem cell therapeutic agent having a specific gene inserted therein, the gene insertion location analysis method comprising:

a basic information management of recording basic information required for operating a gene insertion location analysis system for the stem cell therapeutic agent having the specific gene inserted therein in a DB;
a project starting of registering a consultation process with a customer in a project information DB, registering order information in an order information DB, registering contract conditions based on the order information in a contract information DB, registering project information in a project progress DB, and performing a project;
a sequencing data processing of a data pre-processing for sequencing data generated in the project performing, a data formalization, a data annotation registration, and an analysis data extraction and registration;
a gene insertion location analysis of a search condition setting of setting an analysis target, an analysis method selection, an analysis report confirmation of confirming the analyzed result, and registering an analysis result in a DB; and
a project termination of preparing an analysis report to be submitted to the customer, registering a customer feedback, performing total input cost calculation and billing, and terminating the project.

5. The gene insertion location analysis method of claim 4, wherein the project performing comprises a sequencing operation information registration of registering sequencing operation information in the order information DB and a project progress information DB with respect to the sequencing operation, and

in the sequencing operation information registration,
the sequencing data is read sequentially from beginning to end, and when a last record is read, sequencing operation result information is registered in a gene insertion location information DB and the project progress information DB.

6. The gene insertion location analysis method of claim 5, wherein in the sequencing operation information registration, a record where the sequencing data is a multiple of 4 + 1 is read and then redundant data is registered as one unique value, and variable values are separated and registered in a separate record.

7. The gene insertion location analysis method of claim 5, wherein in the sequencing operation information registration, a record where the sequencing data is a multiple of 4 + 2 is read in units of 4 characters and then the corresponding value is converted to binary number in a nucleic sequence conversion table DB.

8. The gene insertion location analysis method of claim 7, wherein when there is N or U in the values read in units of 4 characters in the sequencing data, or there is no corresponding value in the nucleic sequence conversion table DB, the values are stored in a base information-error table of the gene insertion location information DB.

9.  The gene insertion location analysis method of claim 5, wherein in the sequencing operation information registration, a record where the sequencing data is a multiple of 4 + 4 is compressed as a quality score and then stored in a base information-quality score table of the gene insertion location information DB (45).

10. The gene insertion location analysis method of claim 4, wherein data for analysis of the gene insertion location is generated, analyzed for each subculture, and registered in the gene insertion location information DB, and a correlation between genes by subculture and chromosome is analyzed.

11. The gene insertion location analysis method of claim 10, wherein in the analysis of the gene insertion location, mesenchymal stem cells collected from a bone marrow at the same time and mesenchymal stem cells into which a plurality of genes are inserted are subcultured under the same conditions, and expression level information is comparatively analyzed using a t-value technique.

12. The gene insertion location analysis method of claim 11, wherein the expression level information is comparatively analyze a total expression level by passage, a total number of genes, an expression level by biotype, an expression level by chromosome, the number of genes by chromosome, an expression level by biotype for each chromosome, an expression level by gene, an expression level of the inserted gene, neighboring genes of the inserted gene, and an expression level of the inserted gene.

**FIG. 1**

EP 4 261 829 A1

FIG. 2

10

| 20 | 40 |
|----|----|
| Basic information management module — 21 | Basic information DB — 41 |
| 30 | |
| Order information management module — 31 | Order information DB — 42 |
| Contract information management module — 32 | Contract information DB — 43 |
| Project management module — 33 | Project information DB — 44 |
| Gene insertion location analysis module — 34 | Gene insertion location information DB — 45 |
| Project product management module — 35 | Project progress information DB — 46 |

## FIG. 3A

211 Company information management module
212 User information management module
213 Reference genome information management module
214 COSMIC information management module
215 Equipment information management module
216 Manpower information management module
217 Material information management module
218 Standard operation information management module
219 Nucleic sequence conversion table management module
21a Code information management module
21b Parameter information management module

21

**FIG. 3B**

411 Company information DB
412 User information DB
413 Reference genome information DB
414 COSMIC Information DB
415 Equipment information DB
416 Manpower information DB
417 Material information DB
418 Standard operation information DB
419 Nucleic sequence conversion table DB
41a Code information DB
41b Parameter information DB

41

**FIG. 3C**

Start

Basic information management — 100

Project starting — 200

Sequencing data processing — 300

Gene insertion location analysis — 400

Project termination — 500

End

**FIG. 4**

**FIG. 5**

```
                          ┌─ 200
              ┌──────────────────────┐
              │     Project start     │
              └──────────────────────┘
                          │
                          ▼      ┌─ 210
              ┌──────────────────────┐
              │  Estimate management  │
              └──────────────────────┘
                          │
                          ▼
                   ╱──────────────╲              Failed
                  ╱  Order obtained  ╲─────────────────────────────┐
                  ╲                  ╱                             │
                   ╲──────────────╱                               │
                          │                                        │
                   Succeeded │    ┌─ 220                            │    ┌─ 260
                          ▼                                        ▼
              ┌──────────────────────┐          ┌──────────────────────────┐
              │ Order information     │          │  Failed cause registration │
              │    registration       │          └──────────────────────────┘
              └──────────────────────┘                             │
                          │      ┌─ 230                            │
                          ▼                                        │
              ┌──────────────────────┐                            │
              │ Contract information  │                            │
              │    registration       │                            │
              └──────────────────────┘                            │
                          │      ┌─ 240                            │
                          ▼                                        │
              ┌──────────────────────┐                            │
              │ Project information   │                            │
              │    registration       │                            │
              └──────────────────────┘                            │
                          │      ┌─ 250                            │
                          ▼                                        │
              ┌──────────────────────┐                            │
              │  Project performing   │                            │
              └──────────────────────┘                            │
                          │                                        │
                          ◄────────────────────────────────────────┘
                          ▼
                   (    End    )
```

## FIG. 6

250

Project performing

251

Customer cell information registration

252

DNA extraction operation information registration

253

Library construction information registration

254

Sequencing operation information registration

End

**FIG. 7**

FIG. 8

```
                                    ┌─ 300
              ┌──────────────────────────┐
              │ Sequencing data processing │
              └──────────────────────────┘
                            │
                            ▼                  ┌─ 310
        ┌──────────────────────────────────────┐
        │          Data pre-processing          │
        └──────────────────────────────────────┘
                            │
                            ▼                  ┌─ 320
        ┌──────────────────────────────────────┐
        │           Data formalization          │
        └──────────────────────────────────────┘
                            │
                            ▼                  ┌─ 330
        ┌──────────────────────────────────────┐
        │       Data annotation registration     │
        └──────────────────────────────────────┘
                            │
                            ▼                  ┌─ 340
        ┌──────────────────────────────────────┐
        │  Analysis data extraction and registration │
        └──────────────────────────────────────┘
                            │
                            ▼
                     ╭──────────────╮
                     │     End      │
                     ╰──────────────╯
```

# FIG. 9

310

Data pre-processing

311

Unnecessary data removing

312

Mapping with reference data

313

Redundant data removing

314

Analysis data registration

End

**FIG. 10**

```
                            ┌─ 400
        ┌──────────────────────────────────┐
        │  Gene insertion location analysis  │
        └──────────────────────────────────┘
                        │
                        ▼                ┌─ 410
        ┌──────────────────────────────────────┐
        │        Search condition setting        │
        └──────────────────────────────────────┘
                        │
                        ▼                ┌─ 420
        ┌──────────────────────────────────────┐
        │        Analysis method selection       │
        └──────────────────────────────────────┘
                        │
                        ▼                ┌─ 430
        ┌──────────────────────────────────────┐
        │       Analysis report confirmation     │
        └──────────────────────────────────────┘
                        │
                        ▼                ┌─ 440
        ┌──────────────────────────────────────┐
        │       Analysis result registration     │
        └──────────────────────────────────────┘
                        │
                        ▼
                  (    End    )
```

# FIG. 11

```
                                    ┌─ 500
                        ┌──────────────────────┐
                        │   Project termination │
                        └──────────────────────┘
                                    │
                                    ▼         ┌─ 510
        ┌──────────────────────────────────────────┐
        │          Analysis report submission        │
        └──────────────────────────────────────────┘
                                    │
                                    ▼         ┌─ 520
        ┌──────────────────────────────────────────┐
        │        Customer feedback registration      │
        └──────────────────────────────────────────┘
                                    │
                                    ▼         ┌─ 530
        ┌──────────────────────────────────────────┐
        │      Total input cost calculation and billing │
        └──────────────────────────────────────────┘
                                    │
                                    ▼         ┌─ 540
        ┌──────────────────────────────────────────┐
        │             Project termination            │
        └──────────────────────────────────────────┘
                                    │
                                    ▼
                          (      End      )
```

**FIG. 12**

FIG. 13A

EP 4 261 829 A1

Huffman Coding Method : F41 :1 F5 :1 F30 :2 F1 :4 F1 :1 F8 :1 F6 ,1 F14 ,2 F3 :1 F1 :1 F2 :1 ,1 F3 ,1 F2 ,3 F7 ,1 F1 ,1 F2

151 / 4 => 37 ...3

**FIG. 13B**

EP 4 261 829 A1

EP 4 261 829 A1

```
┌─────────────────────────┐         ┌─────────────────────────┐
│ Entity Group - FastQ    │├───>├───┤│ Project Header          │
└─────────────────────────┘         └─────────────────────────┘
                                       *Project-id,*
                                       *Passage No.*
                                       Created Date,
                                       Modified Date
```

*Project-id,*
*Passage No.*
Created Date,
Modified Date

```
                            ┌─────────────────────────┐              ┌─────────────────────────┐
                         ───┤│ Sequence Data           │├──┤├──○─────┤│ Entity Group – hg38     │
                            └─────────────────────────┘              └─────────────────────────┘
```

*Project-id(char(3))*
*Passage No(char(2))*
*Line No(Var.bit(24))*
Sequence Data(char(151))

```
┌─────────────────────────┐         ┌─────────────────────────┐
│ Sequence Data-sum.      │         │ Sequence Data-Detail    │
└─────────────────────────┘         └─────────────────────────┘
```

| Sequence Data-sum. | Sequence Data-Detail |
|---|---|
| *Project-id(char(3))* | *Project-id(char(3))* |
| *Passage No(char(2))* | *Passage No(char(2))* |
| Lowest X-coordinate(Var.Bit(16)) | *Line No (Var.bit(24))* |
| Highest X-coordinate(Var.Bit(16)) | X-coordinate(Var.Bit(16)) |
| Lowest Y-Coordinate(var.bit(16)) | Y-Coordinate(var.bit(16)) |
| Highest Y-Coordinate(var.bit(16)) | Member of pair(bit(1)) |
| | Filtered(bit(1)) |

## FIG. 14

| Nucleic sequence | Corresponding binary number | Nucleic sequence | Corresponding binary number | Nucleic sequence | Corresponding binary number | Nucleic sequence | Corresponding binary number | Nucleic sequence | Corresponding binary number | Nucleic sequence | Corresponding binary number | Nucleic sequence | Corresponding binary number | Nucleic sequence | Corresponding binary number |
|---|---|---|---|---|---|---|---|---|---|---|---|---|---|---|---|
| AAAA | 0 | AGAA | 100000 | CAAA | 1000000 | CGAA | 1100000 | GAAA | 10000000 | GGAA | 10100000 | TAAA | 11000000 | TGAA | 11100000 |
| AAAC | 1 | AGAC | 100001 | CAAC | 1000001 | CGAC | 1100001 | GAAC | 10000001 | GGAC | 10100001 | TAAC | 11000001 | TGAC | 11100001 |
| AAAG | 10 | AGAG | 100010 | CAAG | 1000010 | CGAG | 1100010 | GAAG | 10000010 | GGAG | 10100010 | TAAG | 11000010 | TGAG | 11100010 |
| AAAT | 11 | AGAT | 100011 | CAAT | 1000011 | CGAT | 1100011 | GAAT | 10000011 | GGAT | 10100011 | TAAT | 11000011 | TGAT | 11100011 |
| AACA | 100 | AGCA | 100100 | CACA | 1000100 | CGCA | 1100100 | GACA | 10000100 | GGCA | 10100100 | TACA | 11000100 | TGCA | 11100100 |
| AACC | 101 | AGCC | 100101 | CACC | 1000101 | CGCC | 1100101 | GACC | 10000101 | GGCC | 10100101 | TACC | 11000101 | TGCC | 11100101 |
| AACG | 110 | AGCG | 100110 | CACG | 1000110 | CGCG | 1100110 | GACG | 10000110 | GGCG | 10100110 | TACG | 11000110 | TGCG | 11100110 |
| AACT | 111 | AGCT | 100111 | CACT | 1000111 | CGCT | 1100111 | GACT | 10000111 | GGCT | 10100111 | TACT | 11000111 | TGCT | 11100111 |
| AAGA | 1000 | AGGA | 101000 | CAGA | 1001000 | CGGA | 1101000 | GAGA | 10001000 | GGGA | 10101000 | TAGA | 11001000 | TGGA | 11101000 |
| AAGC | 1001 | AGGC | 101001 | CAGC | 1001001 | CGGC | 1101001 | GAGC | 10001001 | GGGC | 10101001 | TAGC | 11001001 | TGGC | 11101001 |
| AAGG | 1010 | AGGG | 101010 | CAGG | 1001010 | CGGG | 1101010 | GAGG | 10001010 | GGGG | 10101010 | TAGG | 11001010 | TGGG | 11101010 |
| AAGT | 1011 | AGGT | 101011 | CAGT | 1001011 | CGGT | 1101011 | GAGT | 10001011 | GGGT | 10101011 | TAGT | 11001011 | TGGT | 11101011 |
| AATA | 1100 | AGTA | 101100 | CATA | 1001100 | CGTA | 1101100 | GATA | 10001100 | GGTA | 10101100 | TATA | 11001100 | TGTA | 11101100 |
| AATC | 1101 | AGTC | 101101 | CATC | 1001101 | CGTC | 1101101 | GATC | 10001101 | GGTC | 10101101 | TATC | 11001101 | TGTC | 11101101 |
| AATG | 1110 | AGTG | 101110 | CATG | 1001110 | CGTG | 1101110 | GATG | 10001110 | GGTG | 10101110 | TATG | 11001110 | TGTG | 11101110 |
| AATT | 1111 | AGTT | 101111 | CATT | 1001111 | CGTT | 1101111 | GATT | 10001111 | GGTT | 10101111 | TATT | 11001111 | TGTT | 11101111 |
| ACAA | 10000 | AGAA | 110000 | CCAA | 1010000 | CGAA | 1110000 | GCAA | 10010000 | GGAA | 10110000 | TCAA | 11010000 | TTAA | 11110000 |
| ACAC | 10001 | AGAC | 110001 | CCAC | 1010001 | CGAC | 1110001 | GCAC | 10010001 | GGAC | 10110001 | TCAC | 11010001 | TTAC | 11110001 |
| ACAG | 10010 | AGAG | 110010 | CCAG | 1010010 | CGAG | 1110010 | GCAG | 10010010 | GGAG | 10110010 | TCAG | 11010010 | TTAG | 11110010 |
| ACAT | 10011 | AGAT | 110011 | CCAT | 1010011 | CGAT | 1110011 | GCAT | 10010011 | GGAT | 10110011 | TCAT | 11010011 | TTAT | 11110011 |
| ACCA | 10100 | AGCA | 110100 | CCCA | 1010100 | CGCA | 1110100 | GCCA | 10010100 | GGCA | 10110100 | TCCA | 11010100 | TTCA | 11110100 |
| ACCC | 10101 | AGCC | 110101 | CCCC | 1010101 | CGCC | 1110101 | GCCC | 10010101 | GGCC | 10110101 | TCCC | 11010101 | TTCC | 11110101 |
| ACCG | 10110 | AGCG | 110110 | CCCG | 1010110 | CGCG | 1110110 | GCCG | 10010110 | GGCG | 10110110 | TCCG | 11010110 | TTCG | 11110110 |
| ACCT | 10111 | AGCT | 110111 | CCCT | 1010111 | CGCT | 1110111 | GCCT | 10010111 | GGCT | 10110111 | TCCT | 11010111 | TTCT | 11110111 |
| ACGA | 11000 | AGGA | 111000 | CCGA | 1011000 | CGGA | 1111000 | GCGA | 10011000 | GGGA | 10111000 | TCGA | 11011000 | TTGA | 11111000 |
| ACGC | 11001 | AGGC | 111001 | CCGC | 1011001 | CGGC | 1111001 | GCGC | 10011001 | GGGC | 10111001 | TCGC | 11011001 | TTGC | 11111001 |
| ACGG | 11010 | AGGG | 111010 | CCGG | 1011010 | CGGG | 1111010 | GCGG | 10011010 | GGGG | 10111010 | TCGG | 11011010 | TTGG | 11111010 |
| ACGT | 11011 | AGGT | 111011 | CCGT | 1011011 | CGGT | 1111011 | GCGT | 10011011 | GGGT | 10111011 | TCGT | 11011011 | TTGT | 11111011 |
| ACTA | 11100 | AGTA | 111100 | CCTA | 1011100 | CGTA | 1111100 | GCTA | 10011100 | GGTA | 10111100 | TCTA | 11011100 | TTTA | 11111100 |
| ACTC | 11101 | AGTC | 111101 | CCTC | 1011101 | CGTC | 1111101 | GCTC | 10011101 | GGTC | 10111101 | TCTC | 11011101 | TTTC | 11111101 |
| ACTG | 11110 | AGTG | 111110 | CCTG | 1011110 | CGTG | 1111110 | GCTG | 10011110 | GGTG | 10111110 | TCTG | 11011110 | TTTG | 11111110 |
| ACTT | 11111 | AGTT | 111111 | CCTT | 1011111 | CGTT | 1111111 | GCTT | 10011111 | GGTT | 10111111 | TCTT | 11011111 | TTTT | 11111111 |

FIG. 15A

| Nucleic sequence | Corresponding binary number | Nucleic sequence | Corresponding binary number | Nucleic sequence | Corresponding binary number |
|---|---|---|---|---|---|
| AAAb | 0 | GAAb | 100000 | AAbb | 1000000 |
| AACb | 1 | GACb | 100001 | ACbb | 1000001 |
| AAGb | 10 | GAGb | 100010 | AGbb | 1000010 |
| AATb | 11 | GATb | 100011 | ATbb | 1000011 |
| ACAb | 100 | GCAb | 100100 | CAbb | 1000100 |
| ACCb | 101 | GCCb | 100101 | CCbb | 1000101 |
| ACGb | 110 | GCGb | 100110 | CGbb | 1000110 |
| ACTb | 111 | GCTb | 100111 | CTbb | 1000111 |
| AGAb | 1000 | GGAb | 101000 | GAbb | 1001000 |
| AGCb | 1001 | GGCb | 101001 | GCbb | 1001001 |
| AGGb | 1010 | GGGb | 101010 | GGbb | 1001010 |
| AGTb | 1011 | GGTb | 101011 | GTbb | 1001011 |
| ATAb | 1100 | GTAb | 101100 | TAbb | 1001100 |
| ATCb | 1101 | GTCb | 101101 | TCbb | 1001101 |
| ATGb | 1110 | GTGb | 101110 | TGbb | 1001110 |
| ATTb | 1111 | GTTb | 101111 | TTbb | 1001111 |
| CAAb | 10000 | TAAb | 110000 | Abbb | 1010000 |
| CACb | 10001 | TACb | 110001 | Cbbb | 1010001 |
| CAGb | 10010 | TAGb | 110010 | Gbbb | 1010010 |
| CATb | 10011 | TATb | 110011 | Tbbb | 1010011 |
| CCAb | 10100 | TCAb | 110100 | | |
| CCCb | 10101 | TCCb | 110101 | | |
| CCGb | 10110 | TCGb | 110110 | | |
| CCTb | 10111 | TCTb | 110111 | | |
| CGAb | 11000 | TGAb | 111000 | | |
| CGCb | 11001 | TGCb | 111001 | | |
| CGGb | 11010 | TGGb | 111010 | | |
| CGTb | 11011 | TGTb | 111011 | | |
| CTAb | 11100 | TTAb | 111100 | | |
| CTCb | 11101 | TTCb | 111101 | | |
| CTGb | 11110 | TTGb | 111110 | | |
| CTTb | 11111 | TTTb | 111111 | | |

FIG. 15B

## INTERNATIONAL SEARCH REPORT

| International application No. |
|---|
| **PCT/KR2021/011553** |

**A. CLASSIFICATION OF SUBJECT MATTER**

**G16B 40/20**(2019.01)i; **G16B 30/10**(2019.01)i; **G16B 50/00**(2019.01)i

According to International Patent Classification (IPC) or to both national classification and IPC

**B. FIELDS SEARCHED**

Minimum documentation searched (classification system followed by classification symbols)

G16B 40/20(2019.01); C12Q 1/68(2006.01); G01N 33/15(2006.01); G06Q 10/06(2012.01); G06Q 10/10(2012.01); G16B 30/10(2019.01); G16B 40/00(2019.01); G16B 5/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 유전자 삽입 세포 치료제(gene-cell therapy), 염기서열(nucleic sequence), 분석 (analysis)

**C. DOCUMENTS CONSIDERED TO BE RELEVANT**

| Category* | Citation of document, with indication, where appropriate, of the relevant passages | Relevant to claim No. |
|---|---|---|
| A | KR 10-2006-0015550 A (TAKARA BIO INC.) 17 February 2006 (2006-02-17) See paragraphs [0002], [0070], [0098] and [0101]-[0109] and claim 1. | 1-12 |
| A | KR 10-2056583 B1 (THERAGEN ETEX CO., LTD.) 17 December 2019 (2019-12-17) See paragraphs [0023], [0041], [0101], [0113] and [0148] and claims 1 and 6. | 1-12 |
| A | KR 10-2012-0137235 A (SAMSUNG ELECTRONICS CO., LTD.) 20 December 2012 (2012-12-20) See claim 1. | 1-12 |
| A | KR 10-2011-0057051 A (KIM, Hark Kyun) 31 May 2011 (2011-05-31) See claim 6. | 1-12 |
| A | KR 10-2020-0098189 A (GGULB INC.) 20 August 2020 (2020-08-20) See claim 2. | 1-12 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 December 2021** | **06 December 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

| INTERNATIONAL SEARCH REPORT | International application No. |
|---|---|
| | **PCT/KR2021/011553** |

**C.    DOCUMENTS CONSIDERED TO BE RELEVANT**

| Category* | Citation of document, with indication, where appropriate, of the relevant passages | Relevant to claim No. |
|---|---|---|
| PX | KR 10-2247812 B1 (CELL&BRAIN CO., LTD.) 04 May 2021 (2021-05-04)<br>  See claims 1-12.<br>  *This document is a published earlier application that serves as a basis for claiming<br>  priority of the present international application. | 1-12 |

Form PCT/ISA/210 (second sheet) (July 2019)

**INTERNATIONAL SEARCH REPORT**
Information on patent family members

International application No.

**PCT/KR2021/011553**

| Patent document cited in search report | | | Publication date (day/month/year) | Patent family member(s) | | | Publication date (day/month/year) |
|---|---|---|---|---|---|---|---|
| KR | 10-2006-0015550 | A | 17 February 2006 | CN | 1820081 | A | 16 August 2006 |
| | | | | CN | 1820081 | B | 10 October 2012 |
| | | | | CN | 1820081 | C | 16 August 2006 |
| | | | | EP | 1621638 | A1 | 01 February 2006 |
| | | | | JP | 4496166 | B2 | 07 July 2010 |
| | | | | TW | 200506052 | A | 16 February 2005 |
| | | | | US | 2007-0037139 | A1 | 15 February 2007 |
| | | | | WO | 2004-099446 | A1 | 18 November 2004 |
| KR | 10-2056583 | B1 | 17 December 2019 | None | | | |
| KR | 10-2012-0137235 | A | 20 December 2012 | KR | 10-1969848 | B1 | 17 April 2019 |
| | | | | US | 10090857 | B2 | 02 October 2018 |
| | | | | US | 2013-0031092 | A1 | 31 January 2013 |
| KR | 10-2011-0057051 | A | 31 May 2011 | None | | | |
| KR | 10-2020-0098189 | A | 20 August 2020 | None | | | |
| KR | 10-2247812 | B1 | 04 May 2021 | None | | | |

Form PCT/ISA/210 (patent family annex) (July 2019)